# EUROPEAN PATENT SPECIFICATION

(11) **EP 1 909 766 B1**
(45) Date of publication and mention of the grant of the patent: **16.03.2016**
(21) Application number: 06788707.5
(22) Date of filing: 27.07.2006
(51) Int. Cl.: A61K 9/20, A61K 9/00, A61K 31/55

(54) **PHARMACEUTICAL FORMULATIONS/COMPOSITIONs OF GUANFACINE SUITABLE FOR SINGLE DAILY DOSE**
PHARMAZEUTISCHE FORMULIERUNGEN/ZUSAMMENSETZUNGEN VON GUANFACIN, DIE SICH FÜR EINE TÄGLICHE VERABREICHUNG IN EINZELDOSISFORM EIGNEN
PREPARATIONS/COMPOSITIONS PHARMACEUTIQUES DE GUANFACINE DESTINEES A L'ADMINISTRATION D'UNE DOSE QUOTIDIENNE UNIQUE

(30) Priority: 28.07.2005 US 702982 P
(43) Date of publication of application: 16.04.2008
(73) Proprietor: Shire LLC, Florence, KY 41042 (US)
(72) Inventor: SHOJEAEI, Amir, H., Phoenixville, PA 19460 (US); PENNICK, Michael, Little Milton, Oxford OX44 7QP (GB)
(74) Representative: Atkinson, Jonathan David Mark
(86) International application number: PCT/US2006/029277
(87) International publication number: WO 2007/016284

(56) References cited:
- WO-A-00/41681
- US-A- 5 854 290
- US-B2- 6 811 794

## Description

Guanfacine is useful for treating ADHD as an alternative to stimulant medications, mild to moderate high blood pressure (hypertension), heroin withdrawal, certain problems in difficult pregnancies, sleep disorders, among others. Other indications are being tested. This drug is especially useful in children who have both ADHD and conduct disorder. See USP 6,811,794 and USP 6,287,599 which disclose formulations of guanfacine.

Guanfacine hydrochloride is a centrally acting antihypertensive with α₂-adrenoceptor agonist properties, sold in tablet form for oral administration under the brand name Tenex®. The chemical name of guanfacine hydrochloride is N-amidino-2-(2,6-dichlorophenyl) acetamide hydrochloride and its has the molecular formula, C₉H₁₀Cl₃N₃O. It is in the form of a white to off-white powder that is sparingly soluble in water and alcohol and slightly soluble in acetone, Each commercial tablet for oral administration contains guanfacine hydrochloride equivalent to 1 mg or 2 mg guanfacine. The tablets additionally contain anhydrous lactose, microcrystalline cellulose, povidone, stearic acid and colorants.

Guanfacine may cause the following side effects: dry mouth, constipation, drowsiness, dizziness, headache, difficulty sleeping (insomnia), dry eyes, nausea, vomiting, skin rash or itching, fatigue, indigestion, diarrhea, slow heartbeat, decreased sexual ability, unusual tiredness or weakness, confusion, metal depression, amnesia, liver toxicity, leg cramp, sudden high blood pressure (if stopped abruptly).

For purposes of simplicity, the term "guanfacine" is understood to refer to both the compound guanfacine and any of its salts including guanfacine hydrochloride unless specifically stated otherwise or listed separately from each other.

### The Invention

This invention provides methods, compositions, and formulations that arc useful in the treatment of any of the indications for guanfacine. The methods of the invention include administering to a subject a once a day, oral therapeutic composition or formulation containing guanfacine in the prescribed dose, e.g., 1 mg, 1.5 mg, 2 mg, 2.5 mg, 3mg,3.5 mg, etc., in a single dose form, e.g., a single tablet, which is effective in a once a day regimen and also has a size, e.g., tablet weight, small enough to be acceptable for oral administration, e.g., allowing for easy swallowing by a patient. Accordingly, the compositions and methods of the invention are useful for treating, controlling on affecting ADHD, hypertension, heroin withdrawal, certain problems in difficult pregnancies, sleep disorders, etc., The formulations also have good manufacturability, scalability and robusiness.

An aspect of the invention is a pharmaceutical composition comprising guanfacine and a pharmaceutically acceptable vehicle in a single, once a day discrete dose form for oral administration which is effective in a once a day regimen and also has a size acceptable for patients. For example, typical acceptable sizes, in terms of tablet weight, are for a 1 mg dose, up to 170 mg, for a 2 mg dose, up to 340 mg, for a 2.5 mg dose, up to 255 mg, for a 3 mg dose, up to 225 mg, for a 3.5 mg dose, up to 245 mg, and for a 4 mg dose, up to 300 mg. Sizes may vary with the geometric shape that is acceptable for oral administration, for example.

Round tablets often have diameters up to 0.4 inch (1.016cm), preferably up to 0.35 inch (0.889cm) e.g. 0.1500 inch (0.381cm) to 0.3125 inch (0.794 cm), preferably 0.2000 to 0.3125 inch (0.508cm to 0.794cm), more preferably 0.2813 to 0.3125 inch (0.715cm to 0.794cm) with a thickness of up to 0.2 inch (0.508cm), preferably up to 0.15 inch (0.381cm), e.g., 0.1000 to 0.1400 inch (0.254cm to 0.356cm). If the tablet is oval it often has dimensions of up to 0.6 inch (length) (1.524cm) by up to 0.2 inch (width) (0.508cm) preferably up to 0.55 inch (length) (1.397cm) by up to 0.15 inch (width) (0.381cm) e.g., 0.5400 (length) (1.372cm) by 0.1200 inch (width) (0.305cm), preferably 0.5000 (length) (1.27 cm) by 0.1800 (width) (0.457cm), more preferably 0.4860 (length) (1.234cm) by 0.2400 (width) inch (0.610cm) with a thickness of up to 0.25 inch (0.635cm), preferably up to 0.22 inch (0.559cm), e.g., 0.140 to 0.20 inch (0.356cm to 0.508cm). The shape of the discrete oral dose form can be oval or round or other suitable shape. When the oral dose form, for example, a tablet, is round, the characterizing dimensions usually is its diameter and thickness, and when it is oval, the characterizing dimension is usually its length, width, and thickness.

Another aspect of the invention is such a pharmaceutical composition that has a total weight of, for example, up to about 340 mg, e.g., 100 to 300mg, preferably 130 to 300mg, more preferably 150 to 300mg.

In a further aspect the preferred tablet of the invention has a hardness of 4.0 (39.23N) to 11.0 kp (107.87N), preferably 4.5 to 9.5 kp (44.13N to 93.16N), more preferably 4.5 to 8.0 kp (44.13N to 78.45N), measured using a Schleuniger tablet hardness tester.

Another aspect is a method for the treatment of ADHD with the pharmaceutical composition described above, for example, in adults (18+ years), and children, e.g., about ages 6-12 and about ages 13-17. Thus, this invention includes tablets that are able to achieve an effective pk profile, and size, e.g., weight. In another aspect, the tablets also achieve sufficient compressibility to attain desired tablet hardness values.

Advantages of being able to administer guanfacine in a single once a day dose form include lessening the chance for a patient missing a prescribed administration regimen, e.g., no chance of forgetting taking a pill twice, three times, etc., daily; case of administration to a child daily versus more than once if the child resists or generally objects to taking medication each time administered; loss interference with daily activities, e.g., school, sports practice etc. for kids, work, etc.; and convenience; etc.

Another aspect of the invention is the pharmaceutical formulation of the invention having a mean plasma concentration pharmacokinetic (PK) profile such that, when the formulation is administered in a dose of 1 mg of guanfacine to fasted healthy adult subjects, said PK profile has at least one of the following parameters: an AUC_{0-Inst} of about 29.3 ± 8.8 ng·h/mL, an AUC_{∞} of about 32.4 ± 8.8 ng·h/mL, a mean Cₘₐₓ of about 0.98 ± 0.26 ng/mL, and a median Tₘₐₓ of about 6 hours (each parameter. being within ±50%, more preferably ± 40% and particularly preferably ± 20% of said respective value (irrespective of stated ± value), and when the formulation is administered in a dose amount of 2 mg or another dose amount whose PK profile is linearly correlated with that of a 1 mg dose, an AUC₀₋ₗₐₛₜ, AUC_{∞}, and Cₘₐₓ linearly related to those for the 1 mg dose. The t_{1/2}, measured for the 1 mg formulation, is 17.5 ± 3.8 hours.

Another aspect of the invention is the pharmaceutical formulation of the invention having a mean plasma concentration pharmacokinetic (PK) profile such that, when the formulation is administered in a dose of 2.5 mg of guanfacine to fasted healthy adult subjects, said PK profile has at least one of the following parameters: an AUC₀₋ₗₐₛₜ of about 81.3 ± 35.4 ng·h/mL, an AUC_{∞} of about 85.0 ± 37.4 ng·h/mL, a mean Cₘₐₓ of about 2.49 ± 0.93 ng/mL, and a median Tₘₐₓ of about 6 hours (each parameter being within ± 50%, more preferably ± 40% and particularly preferably ± 20% of said respective value (irrespective of stated ± value), and when the formulation is administered in a dose amount whose PK profile is linearly correlated with that of a 2.5 mg dose, an AUC₀₋ₗₐₛₜ, AUC_{∞}, and Cₘₐₓ linearly related to those for the 2.5 mg dose. The t_{1/2}, measured for.the 1 mg formulation, is 16.7 ± 7.4 hours.

Another aspect of the invention is the pharmaceutical formulation of the invention having a mean plasma concentration pharmacokinetic (PK) profile such that, when the formulation is administered in a dose of 4 mg of guanfacine to fasted healthy adult subjects, said PK profile has at least one of the following parameters: an AUC₀₋ₗₐₛₜ of about 120 ± 41.5 ng·h/mL, an AUC_{∞} of about 125 ± 46.0 ng·h/mL, a mean Cₘₐₓ of about 3.58 ± 1.39 ng/mL, and a median Tₘₐₓ of about 5 hours (each parameter being within ± 50%, more preferably ± 40% and particularly preferably ± 20% of said respective value (irrespective of stated ± value), and when the formulation is administered in a dose amount of 3 mg, 3.5 mg or another dose amount whose PK profile is linearly correlated with that of a 4 mg dose, an AUC₀₋ₗₐₛₜ, AUC_{∞}, and Cₘₐₓ linearly related to those for the 4 mg dose. The t_{1/2}, measured for the 1 mg formulation, is 17.1 ± 5.5 hours.

Another aspect of the invention is the pharmaceutical formulation of the invention having a mean plasma concentration pharmacokinetic (PK) profile such that, when the formulation is administered in a dose of 2 mg of guanfacine to fasted patients ages 6-12 suffering from ADHD, said PK profile has at least one of the following parameters: an AUC₀₋ₗₐₛₜ of about 56.88 ± 22.05 ng·h/mL, an AUC_{∞} of about 65.20 ± 23.88 ng·h/mL, a mean Cₘₐₓ of about 2.55 ± 1.03 ng/mL, and a median Tₘₐₓ of about 5 hours (each parameter being within ± 50%, more preferably ± 40% and particularly preferably ± 20% of said respective value (irrespective of stated ± value), and when the formulation is administered in a dose amount of 1 mg or another dose amount whose PK profile is linearly correlated with that of a 2 mg dose, an AUC₀₋ₗₐₛₜ, AUC_{∞}, and Cₘₐₓ linearly related to those for the 2 mg dose. The t_{1/2}, measured for the 2 mg formulation, is 14.4 ± 2.4 hours.

Another aspect of the invention is the pharmaceutical formulation of the invention having a mean plasma concentration pharmacokinetic (PK) profile such that, when the formulation is administered in a dose of 2 mg of guanfacine to fasted patients ages 13-17 suffering from ADHD, said PK profile has at least one of the following parameters: an AUC₀₋ₗₐₛₜ of about 42.74 ± 12.85 ng·h/mL, an AUC_{∞} of about 47.25 ± 13.69 ng·h/mL, a mean Cₘₐₓ of about 1.69 ± 0.43 ng/mL, and a median Tₘₐₓ of about 5 hours (each parameter being within ± 50%, more preferably ± 40% and particularly preferably ± 20% of said respective value (irrespective of stated ± value), and when the formulation is administered in a dose amount of 1 mg or another dose amount whose PK profile is linearly correlated with that of a 2 mg dose, an AUC₀₋ₗₐₛₜ, AUC_{∞}, and Cₘₐₓ linearly related to those for the 2 mg dose. The t_{1/2}, measured for the 2 mg formulation, is 17.9 ±5.8 hours.

Formulations of the invention contain 0.75-2% (w/w) of guanfacine. Amounts of other ingredients will vary with the formulation technology used and the PK profiles to be achieved, as will the amount of guanfacine, all as shown herein.

All patients can get any of the 1, 2, 2.5, 3, 3.5 or 4 mg once a day formulations based on desired PK profiles and effectiveness to be achieved. All patients could get the 3 mg dose (if they are not adequately maintained on lower doses). The dose for more mature patients, e.g., teenagers and adults, and also for heavier children may be titrated to 3.5 or 4mg, for example.

Preferred formulations that achieve the desired PK profile are provided in Table 1. More preferred formulations that achieve the desired PK profile are provided in Table 2.

**TABLE 1:**

| Component | 1 mg Formulation | | 2 mg Formulation | | 2.5 mg Formulation | | 3 mg Formulation | | 3.5 mg Formulation | | 4 mg Formulation | |
|---|---|---|---|---|---|---|---|---|---|---|---|---|
| | **mg/tablet** | **%w/w** | **mg/tablet** | **%w/w** | **mg/tablet** | **%w/w** | **mg/tablet** | **%w/w** | **mg/tablet** | **%w/w** | **mg/tablet** | **%w/w** |
| Guanfacine Hydrochloride, USP/NF | 1.14 | 0.76-0.88 | 2.28 | 0.76-0.88 | 2.85 | 1.27-1.42 | 3.42 | 1.71-1.90 | 3.99 | 1.71-1.90 | 4.56 | 1.71-1.90 |
| Hypromellose 2208, USP/NF (Methocel^{®} K4M CR Premium) | 17.34-20.01 | 13.34 | 34.68-40.02 | 13.34 | 26.68-30.02 | 13.34 | 18.00-20.00 | 10.00 | 21.00-23.33 | 10.00 | 24.00-26.67 | 10.00 |
| High Density Silicified Microcrystalline Cellulose (Prosolv HD90™) | 20.00-26.00 | 15.38-17.33 | 40.00-51.99 | 15.38-17.33 | 34.19-38.99 | 17.10-17.33 | 37.88-42.78 | 21.04-21.39 | 44.18-49.91 | 21.04-21.39 | 50.49-57.04 | 21.04-21.39 |
| Lactose Monohydrate, Povidone, Crospovidone Granulated Blend (Ludipress^{®}) | 22.02 - 25.37 | 16.91 - 16.94 | 44.04-50.73 | 16.91 - 16.94 | 31.98-35.78 | 15.90-15.99 | 38.80 - 42.80 | 21.40 21.55 | 45.25 - 49.93 | 21.40 - 21.55 | 51.72- 57.07 | 21.40 21.55 |
| Methacrylic Acid Copolymer, Type C, USP/NF (Eudragit^{®} L 100-55) | 45.00-50.00 | 33.33-34.61 | 90.00-99.99 | 33.33-34.61 | 66.66-74.99 | 33.33 | 45.00-50.00 | 25.00 | 52.50-58.33 | 25.00 | 60.00-66.67 | 25.00 |
| Fumaric Acid, FCC | 6.50 - 7.50 | 5.00 | 13.00-15.00 | 5.00 | 10.00-11.25 | 5.00 | 9.00-10.00 | 5.00 | 10.50-11.67 | 5.00 | 12.00-13.33 | 5.00 |
| Atomized Glyceryl Behenate, USP/NF (Compritol^{®} 888 ATO) | 18.00-20.00 | 13.33-13.85 | 36.00-39.99 | 13.33-13.85 | 26.66-29.99 | 13.33 | 27.00-30.00 | 15.00 | 31.50-35.00 | 15.00 | 36.00-40.00 | 15.00 |
| Pigment Blend Powder* | - | - | - | - | 1.00-1.13 | 0.50 | 0.90-1.00 | 0.50 | 1.05-1.17 | 0.50 | 1.20 -1.33 | 0.50 |
| **Total** | **130.00-150.00** | **100.00** | **260.00-300.00** | **100.00** | **200.00-225.00** | **100.00** | **180.00-200.00** | **100.00** | **210.00-233.33** | **100.00** | **240.00-266.66** | **100.00** |
| **Tablet Size/Shape** | 9/32" | Round | 0.486" x 0.240" | Oval | Round | 5/16" | Round | 5/16" | Round | 5/16" | 0.486" x 0.240" | Oval |

| | | | | | | | | | | | | |
|---|---|---|---|---|---|---|---|---|---|---|---|---|
| *2.5 mg: Blue pigment; 3 mg: Green pigment; 3.5 mg: Red pigment; 4 mg: Green pigment | | | | | | | | | | | | |

**TABLE 2:**

| Component | 1 mg Formulation | | 2 mg Formulation | | 2.5 mg Formulation | | 3 mg Formulation | | 3.5 mg Formulation | | 4 mg Formulation | |
|---|---|---|---|---|---|---|---|---|---|---|---|---|
| | **mg/tablet** | **%w/w** | **mg/tablet** | **%w/w** | **mg/tablet** | **%w/w** | **mg/tablet** | **%w/w** | **mg/tablet** | **%w/w** | **mg/tablet** | **%w/w** |
| Guanfacine Hydrochloride, USP/NF | 1.14 | 0.76 | 2.28 | 0.76 | 2.85 | 1.27 | 3.42 | 1.71 | 3.99 | 1.71 | 4.56 | 1.71 |
| Hypromellose 2208, USP/NF (Methocel^{®} K4M CR Premium) | 20.01 | 13.34 | 40.02 | 13.34 | 30.02 | 13.34 | 20.00 | 10.00 | 23.33 | 10.00 | 26.67 | 10.00 |
| High Density Silicified Microcrystalline Cellulose (Prosolv HD90™) | 26.00 | 17.33 | 51.99 | 17.33 | 38.99 | 17.33 | 42.78 | 21.39 | 49.91 | 21.39 | 57.04 | 21.39 |
| Lactose Monohydrate, Povidone, Crospovidone Granulated Blend (Ludipress^{®}) | 25.37 | 16.91 | 50.73 | 16.91 | 35.78 | 15.90 | 42.80 | 21.40 | 49.93 | 21.40 | 57.07 | 21.40 |
| Methacrylic Acid Copolymer, Type C, USP/NF (Eudragit^{®} L 100-55) | 50.00 | 33.33 | 99.99 | 33.33 | 74.99 | 33.33 | 50.00 | 25.00 | 58.33 | 25.00 | 66.67 | 25.00 |
| Fumaric Acid, FCC | 7.50 | 5.00 | 15.00 | 5.00 | 11.25 | 5.00 | 10.00 | 5.00 | 11.67 | 5.00 | 13.33 | 5.00 |
| Atomized Glyceryl Behenate, USP/NF (Compritol^{®} 888 ATO) | 20.00 | 13.33 | 39.99 | 13.33 | 29.99 | 13.33 | 30.00 | 15.00 | 35.00 | 15.00 | 40.00 | 15.00 |
| Pigment Blend Powder* | --- | --- | --- | --- | 1.13 | 0.50 | 1.00 | 0.50 | 1.17 | 0.50 | 1.33 | 0.50 |
| **Total** | **150.00** | **100.00** | **300.00** | **100.00** | **225.00** | **100.00** | **200.00** | **100.00** | **233.33** | **100.00** | **266.66** | **100.00** |
| **Tablet Size/Shape** | 9/32" | Round | 0.486" x 0.240" | Oval | 5/16" | Round | 5/16" | Round | 5/16" | Round | 0.486" x 0.240" | Oval |
| **Tablet Thickness** | 0.120" | ------ | 0.200" | ------ | 0.140" | ------ | 0.130" | ------ | 0.140" | ------ | 0.170" | ------ |

| | | | | | | | | | | | | |
|---|---|---|---|---|---|---|---|---|---|---|---|---|
| *2.5 mg: Blue pigment; 3 mg: Green pigment; 3.5 mg: Red pigment; 4 mg: Green pigment | | | | | | | | | | | | |

Further aspects of the invention include formulations which combine two or more of the pharmacokinetic parameters, or sets of pharmacokinetic parameters (e.g., AUC₀₋ₗₐₛₜ, AUC_{∞}, Cₘₐₓ and/or Tₘₐₓ), described above, for example two, three, or four, etc., pharmacokinetic parameters. For example, such aspects include formulations which provide one or more of the pharmacokinetic parameters, or sets thereof, as shown herein when administered to adult patients and one or more of the pharmacokinetic parameters, or sets thereof, as shown herein when administered to children ages 6-12 and/or children ages 13-17. Preferred combinations of two or more pharmacokinetic parameters to be satisfied are AUC₀₋ₜ and Cₘₐₓ.

The formulations of this invention are useful over an extended period. By "extended period" is meant that the formulation of guanfacine of this invention can be administered to a patient for a length of time of about a week, a month, 2-11 months, (e.g., 2-6 months, 7-11 months, etc.), a year, two or more years, etc. and continue to be effective to treat the indication involved, e.g., ADHD.

In an advantageous embodiment, the pharmaceutical formulations of guanfacine comprise Eudragit L® 100-55 (poly(methacrylic acid, ethyl acrylate)), (anionic polymer of methacrylic acid and methacrylates - Methacrylic copolymer Type C, NF) marketed by Rohm America, Inc. The compositions in this embodiment contain Eudragit L® 100-55, out of one hundred percent by weight of total composition (w/w), from about 25 percent to about 45 percent, preferably from about 25 percent to about 35 percent, most preferably from about 26, 27, 28, etc. percent to about 32, 33, 34, 35, etc. percent (w/w) of the total composition, the content optionally varying with dosage level as shown below.

A further aspect includes formulations which achieve the PK profile described above using formulations based on conventional galenical technology using osmotic release, pulsatile release, sustained release, controlled release, delayed release, extended release, immediate release or other modified release of guanfacine.

In other aspects, the invention is directed to methods for treatment using, as described above, guanfacine provided in a modified release formulation. The formulation is administered to an adult patient (18+ years), about 6-12 year olds, or about 13-17 year olds and achieves treatment of ADHD, hypertension, heroin withdrawal, certain problems in difficult pregnancies, sleep disorders, etc.

In yet another aspect, the invention pertains to a packaged pharmaceutical composition or formulation comprising a container holding a pharmaceutical composition comprising guanfacine and instructions for using the compound for an indication for guanfacine, e.g., ADHD, hypertension, heroin withdrawal, certain problems in difficult pregnancies, sleep disorders in a subject, etc.

The term "vehicle" has the broadest meaning, including any substance with which drugs are formulated.

The term "container" includes any receptacle for holding the therapeutic formulation, For example, in one embodiment, the container is the packaging that contains the formulation. In other embodiments, the container is not the packaging that contains the formulation, *i.e*., the container is a receptacle, such as a box or vial that contains the packaged formulation or unpackaged formulation and the instructions for use of the formulation. Moreover, packaging techniques are well known in the art. It should be understood that the instructions for use of the therapeutic formulation may be contained on the packaging containing the therapeutic formulation, and as such the instructions form an increased functional relationship to the packaged product. However, it should be understood that the instructions can contain information pertaining to guanfacine's ability to perform its intended function, *e.g*., treat ADHD, hypertension, heroin withdrawal, certain problems in difficult pregnancies, sleep disorders, etc.

Supplementary active compounds can also be incorporated into the compositions as long as they do not significantly affect the ability of the therapeutic formulation to perform its intended function or do not significantly affect the ability of the therapeutic formulation to achieve the intended plasma concentration pharmacokinetic (PK) profile as described above.

In certain embodiments of the invention, the subject is in need of treatment by the methods of the invention, and is selected for treatment based on this need. A subject in need of treatment is art-recognized, and includes subjects that have been identified as having a disease or disorder related to ADHD, hypertension, heroin withdrawal, certain problems in difficult pregnancies, sleep disorders, etc., having a symptom of such a disease or disorder, or at risk of such a disease or disorder, and would be expected, based on diagnosis, *e.g*., medical diagnosis, to benefit from treatment (*e.g*., curing, healing, preventing, alleviating, relieving, altering, remedying, ameliorating, improving, or affecting the disease or disorder, the symptom of the disease or disorder, or the risk of the disease or disorder).

Administration of guanfacine to a subject to be treated can be carried out using known procedures, at dosages and for periods of time effective to achieve the intended treatment in the subject. An effective amount of the therapeutic compound necessary to achieve a therapeutic effect may vary according to factors such as the age, sex, and weight of the subject. Actual dosage levels of guanfacine in the pharmaceutical compositions of this invention may be varied so as to obtain an amount of the active ingredient that is effective to achieve the desired therapeutic response for a particular patient, composition, and mode of administration, without being toxic to the patient.

Depending on the formulation system to be used, pharmaceutically acceptable carriers include any of the following: fillers or extenders, such as sugars, starches, lactose, sucrose, glucose, mannitol, or silicic acid; binders, such as, for example, carboxymethylcellulose, alginates, gelatin, polyvinyl pyrrolidone, sucrose or acacia; humectants, such as glycerol; disintegrating agents, such as agar-agar, calcium carbonate, potato or tapioca starch, alginic acid, certain silicates, sodium starch glycolate, cross-linked sodium carboxymethyl cellulose, cross-linked sodium carboxymethyl cellulose (e.g., AC-DI-SOL®), sodium starch glycolate (e.g., EXPLOTAB®, PRIMOJEL®), and cross-linked polyvinylpolypyrrolidone (e.g., Plasone-XL), and sodium carbonate; solution retarding agents, such as paraffin; absorption accelerators, such as quaternary ammonium compounds; excipients, such as cocoa butter; solvents; encapsulating material; wetting agents, such as, for example, cetyl alcohol and glycerol monostearate; absorbents, such as kaolin and bentonite clay; lubricants such as talc, stearic acid, leucine, glyceryl behanate, hydrogenated vegetable oil, calcium stearate, magnesium stearate, solid polyethylene glycols, sodium lauryl sulfate; coloring agents; preservatives; surface-active agents; dispersing agent; inert liquid diluent; adjuvants; emulsifying agents; coating agents; sweetening, flavoring and/or perfuming agents; suppository waxes; oils, such as peanut oil, cottonseed oil, safflower oil, sesame oil, olive oil, corn oil and soybean oil; antioxidants, for example, water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; sodium citrate; dicalcium. phosphate; phosphate buffer solutions; and other non-toxic compatible substances employed in pharmaceutical formulations known in the art. Prevention of the action of microorganisms on the tablets may be ensured by the inclusion of various antibacterial and antifungal agents, for example, paraben, chlorobutanol, phenol sorbic acid, and the like. Each carrier must be "acceptable" in the sense of being compatible with the other ingredients of the formulation; not injurious to the patient, and in the sense that it does not affect the ability of the therapeutic formulation to achieve desired PK profiles.

As noted above, formulations for achieving the foregoing PK profiles dosing regimens can use immediate, controlled, sustained, extended, delayed, pulsatile, osmotic release, etc. technologies, alone or in combination to achieve the desired results. Examples of principles of formulations and preparations are contained, for example, in the Handbook of Pharmaceutical Excipents, American Pharmaceutical Association 4th edition, 2003 (Rowe, Sheskey and Weller); Pharmaceutical Dosage Forms: Tablets (Lieberman, Lachman and Schwarts, editors) current edition, published by Marcel Dekker, Inc., as well as Remington's Pharmaceutical Sciences (A. Gennaro, editor, 20th edition, 2000). For conventional sustained or controlled release methods, see, e.g., in R.K. Chang and J.R. Robinson, chapter 4: "Sustained Drug Release from Tablets and Particles Through Coating," in Pharmaceutical Dosage Forms: Tablets, volume 3, edited by H.A. Lieberman, L. Lachman, and J.B. Schwartz, Marcel Dekker, Inc., 1991; R.J. Campbell and G.L. Sackett, chapter 3: "Film coating," in Pharmaceutical Unit Operations: Coating, edited by K.E. Avis, A.J. Shukla, and R.K. Chang, Interpharm Press, Inc., 1999. Principles of alternative formulation techniques are also given in US patents 6,878,386; 6,849,661; 6,827,947; 6,702,803; 6,793,936; 6,780,436; 6,746,692; 6,638,535; 6,635,277; 6,627,223.

Other guanfacine formulations different from those specifically exemplified herein can be provided in the form of beads, e.g., having a core which is optionally coated with a coating which allows the release of the agent immediately or over time, such as a pharmaceutically a cceptable water-insoluble or water soluble film former alone or with a dissolution regulating agent etc.. See, e.g., USP 4,728,512. A biphasic or multiphasic release profile can be achieved by combining the immediate-release beads with delayed, sustained or other controlled release beads or by providing various extended release beads with differing release profiles - all to achieve the desired PK profiles.

Beads can be prepared by coating conventional drug-containing cores with a water-insoluble polymer, or a combination of water-insoluble polymers, or a combination of water-insoluble and water-soluble polymers. This may be a combination of layers, or a combination of polymers in a single coating. The resultant beads (or tiny tablets) can then be placed in a capsule. Other than beads in a capsule shell, tablets in a capsule shell (e.g., one or more immediate-release tablet and one or more delayed, sustained release tablet in a capsule shell) also can be used to attain the desired release profile.

Various polymeric materials can be used to achieve the desired type of pattern of release, e.g., immediate, sustained, delayed etc. release. For example, a multiple dosage form (e.g., as discussed below) can deliver rapid and complete dosages of active agent to a recipient multiple times over a period of hours with a single oral administration, if it is desired to combine dosages in a single unit; additionally, doses with sustained or delayed release function can be combined with each other or with doses having immediate release functionality.

Examples of possible bead constructions are plentiful and include the following:
- Sugar core bead, coated with active agent and then coated with polymer and/or with mix of active agent and polymer or any different order of such layers on the core, within each case, selected active agent concentrations of components in the layers.
- Bead containing active agent core, coated with polymer, and/or with mix of active agent and polymer or any different order of such layers on the core, within each case, selected active agent concentrations of components in the layer.
- Tablet or capsule containing multiple types of beads as described above having differing timing of release or different rates of release of active agent.

Matrix beads can also be used, i.e., not having any layers to achieve sustained or delayed release. The components used in such matrices are usually chosen from conventional sustained release or delayed release polymers.

Guanfacine-containing particles may also be incorporated into a tablet, in particular by incorporation into a tablet matrix, which rapidly disperses the particles after ingestion, in certain embodiments. In order to incorporate these particles into such a tablet, a filler/binder is added to a tablet that can accept the particles, but will not allow their destruction during the tableting process. Materials that are suitable for this purpose include, but are not limited to, microcrystalline cellulose (e.g., AVICEL®), soy polysaccharide (e.g., EMCOSOY®), pre-gelatinized starches (e.g., STARCH® 1500, NATIONAL® 1551), and polyethylene glycols (e.g., CARBOWAX®). The materials are typically present in the range of 5-75% (w/w), with a preferred range generally of 25-50% (w/w).

Various enteric materials, e.g., cellulose acetate phthalate, hydroxypropyl methylcellulose phthalate, polyvinyl acetate phthalate, and the EUDRAGIT® and ACRYLEZE® acrylic polymers, can also be employed. These are gastroresistant, enterosoluble coatings for drug release in the intestine when desired. The enteric materials, which are soluble at higher pH values, are frequently used for colon-specific delivery systems and are entirely conventionally employable in the systems of this invention. The enteric polymers used in this invention can also be modified conventionally by mixing with other known coating products that are not pH sensitive. Examples of such coating products include the neutral methacrylic acid esters with a small portion of trimethylammonioethyl methacrylate.chloride, which are commercially available, e.g., EUDRAGIT® RS and EUDRAGIT® RL; neutral ester dispersions without any functional groups, e.g., EUDRAGIT® NE30D and EUDRAGIT® NE30; most preferably, EUDRAGIT® L100-55, and other pH independent coating products.

A conventional protective coating layer may also be applied immediately outside the core, either a drug-containing matrix core or a drug-layered core, by conventional coating techniques such as pan coating or fluid bed coating using solutions of polymers in water or suitable organic solvents or by using aqueous polymer dispersions. Suitable materials for the protective layer include cellulose derivatives such as hydroxyethyl cellulose, hydroxypropyl cellulose, hydroxypropyl methylcellulose, polyvinylpyrrolidone, polyvinylpyrrolidone/vinyl acetate copolymer, ethyl cellulose aqueous dispersions, polyvinyl acetate (e.g., AQUACOAT®, SURELEASE®), EUDRAGIT®'s, OPADRY® and the like. Typical coating levels are from 1 to 6%, in general, preferably 2-4% (w/w).

An overcoating layer can further optionally be applied to the compositions of the present invention. OPADRY®, OPADRY II® (Colorcon) and corresponding color and colorless grades from Colorcon can be used to protect the pellets from being tacky and provide colors to the product. Typical levels of protective or color coating are from 1 to 6%, in general preferably 2-3% (w/w). Many ingredients can be incorporated into the overcoating formula, for example to provide a quicker (immediate) release, such as plasticizers: acetyltriethyl citrate, triethyl citrate, acetyltributyl citrate, dibutylsebacate, triacetin, polyethylene glycols, propylene glycol and the others; lubricants: talc, colloidal silica dioxide, magnesium stearate, calcium stearate, titanium dioxide, magnesium silicate, and the like.

Optional modifying components of a protective layer which can be used over the enteric or other coatings include a water penetration barrier layer (semi-permeable polymer) which can be successively coated after the enteric or other coating to reduce the water penetration rate through the enteric coating layer and thus increase the lag time of the drug release. Sustained-release coatings commonly known to one skilled in the art can be used for this purpose by conventional coating techniques such as pan coating or fluid bed coating using solutions of polymers in water or suitable organic solvents or by using aqueous polymer dispersions. For example, the following materials can be used, but not limited to: cellulose acetate, cellulose acetate butyrate, cellulose acetate propionate, ethyl cellulose, fatty acids and their esters, waxes, zein, and aqueous polymer dispersions such as EUDRAGIT®'s, e.g., RS, RL 30D, NE 30D, AQUACOAT®, SURELEASE®, cellulose acetate latex, etc. Combinations of the above polymers and hydrophilic polymers such as hydroxyethyl cellulose, hydroxypropyl cellulose (KLUCEL®, Hercules Corp.), hydroxypropyl methylcellulose (METHOCEL®, Dow Chemical Corp.), and polyvinylpyrrolidone can also be used.

Details of using the foregoing constructs and others to achieve a desired release profile as discussed above are fully conventional and can be determined by those of skill in the art with at most a few routine parametric experiments, and conventional adjustments, e.g., involving identities of polymers and mixtures thereof, relative amounts of components, coating thicknesses, bead diameters, number of layers and compositions thereof, etc. Thus, for example, for a given construct, in vitro dissolution profiles can be determined. Fully conventional formulation and dissolution profile adjustments can be made routinely. Formulations having the desired in vitro release profiles produce the desired plasma concentration levels. These plasma profiles will be correlated with the in vitro release profiles in view of the usual factors, e.g., in vivo dissolution and absorption properties, active agent half-lives, etc., which correlation is well understood in the art.

Suitable materials which can be used to achieve formulations having such release profiles are well known and include but are not limited to polyvinyl acetate, cellulose acetate, cellulose acetate lattices, cellulose acetate butyrate, cellulose acetate propionate, ethyl cellulose, fatty acids and their esters, alkyl alcohols, waxes, zein (prolamine from corn), and aqueous polymeric dispersions such as the commercially available Eudragit^{®}, Aquacoat^{®}, Surelease^{®}, Kollicoat^{®}, etc., products.

The tablets (and other possible solid dosage forms of the pharmaceutical compositions of the present invention, such as dragees, capsules, pills, pellets, and granules,) may optionally be scored.

In general, the pharmaceutical formulations comprise 0.1 to 40% by weight of any single ingredient, more typically 0.5-35% by weight, depending on the formulation technology employed.

### Definition of terms

- Cₘₐₓ -: the maximum observed plasma concentration.
- Tₘₐₓ -: the time of occurrence of Cₘₐₓ.
- AVC₀₋ₗₐₛₜ -: the area under the plasma concentration versus time curve from time zero to the last sampling time at which concentrations were at or above the limit of quantification, calculated by the linear trapezoidal rule.
- AUC_{∞} -: the area under the plasma concentrations versus time curve from time zero to infinity, calculated from AUC₀₋ₜ⁺ (Cₗₐₛₜ/λ_{z}), where Cₗₐₛₜ is the last observed quantifiable concentration and λz is the apparent terminal rate constant.
- t_{1/2} -: the apparent terminal half-life, calculated from 1n2/λ_{z}.
- API -: Active pharmaceutical ingredient
- Fasted -: Overnight fasting, e.g., usually about 10-12 hours.

### Brief Description of the Drawings

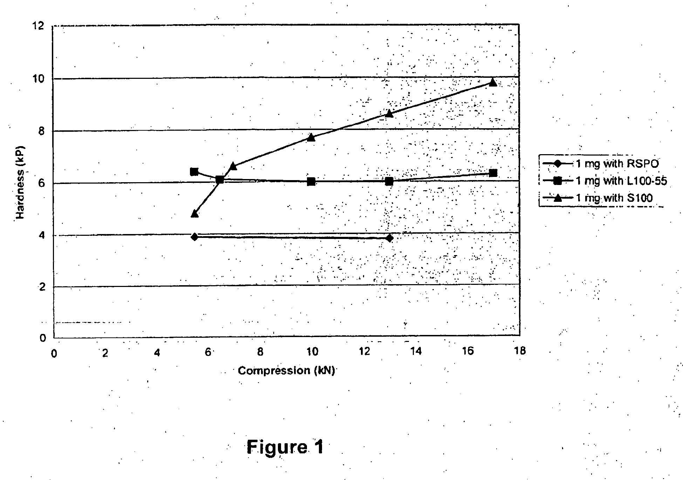

- Figure 1: shows the range of hardness at different compression forces for formulations with different Eudragit polymers.

### Examples

The invention is further illustrated by the following examples which should not be construed as further limiting the subject invention.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing and in the examples, all temperatures are set forth uncorrected in degrees Celsius and, all parts and percentages are by weight, unless otherwise indicated. The particle size range of the guanfacine HCl (as the API) used in the foregoing examples, tables and figures, is dv50 of 5microns to 25 microns and dv90 of 10 microns to 50 microns. The term "dv" refers to volume diameter (the diameter of a sphere having the same volume) and 50 means 50 percent of the particles and 90 means 90 percent of the particles

### Example 1

The primary pieces of equipment used for manufacturing the tablets are a 16 quart V-shaped bleeder equipped with an intensifier bar and a 16 station rotary tablet press. All materials are passed through a 40 mesh screen and charged into a 16 quart V-blender, with guanfacine sandwiched in the middle. The mix is blended for ten minutes, with the intensifier bar turned on for minutes 5 - 8. The blend is charged into a polyethylene bag and then transferred to the hopper of the Stokes tablet press. The blend is compressed to the appropriate hardness for the necessary tablet weight. Tablet hardness is tested with a Schleuniger hardness tester.

Formulations containing either Eudragit L100-55 or Eudragit RSPO were tested for hardness at various compression forces (see Figure 1).

| **Ingredients** | **Composition 1 (% w/w)** | **Composition 2 (% w/w)** |
|---|---|---|
| Guanfacine HCl | 0.76 | 0.76 |
| Methocel K4M* | 13.34 | 13.34 |
| Eudragit L100-55* | 33.33 | N/A |
| Eudragit RSPO | N/A | 33.33 |
| Pumaric acid | 5.00 | 5.00 |
| Compritol 888 ATO* | 13.33 | 13.33 |
| Ludipress* | 16.91 | 16.91 |
| Prosolv HD90* | 17.33 | 17.33 |

| | | |
|---|---|---|
| *Methocel K4M -Hydroxypropylmethylcellulose, Dow Chemical; Eudragit L100-55-poly(methacrylic acid, ethyl acrylate), a methacrylic acid copolymer, Rohm America, Inc.; Eudragit RSPO - Ammonio Methacrylate copolymer, Rohm America, Inc.; Compritol 888 ATO- Glyceryl dibehenate, Gattefosse; Ludipress- Lactose/povidonc/crospovidone, BASF; Prosolv HD90 - Silicified microcrystalline cellulose, Penwest. | | |

| Composition | Average Hardness (kP) | Average Hardness (N) |
|---|---|---|
| 1 (Eudragit L100-55) | 6.2 | 60.80 |
| 2 (Eudragit RSPO) | 3.9 | 38.25 |

The general method for preparing Guanfacine tablets used herein is similar to the method for the same in US 6,811,794 and US 6,287,599.

### Example 2

Objective: The objective of this study was to assess the pharmacokinetics of controlled-release guanfacine after a single 2 mg dose or multiple doses of 2 mg and 4 mg from Table 2.

Methods: An open-label, dose-escalation study in male and female children (aged 6-12 years) and adolescents (aged 13-17 years) with ADHD. Subjects weighed 25 kg (55 1b) or more but were not considered overweight in the opinion of the investigator. Their electrocardiogram readings (ECGs) were within normal ranges, and their blood pressure measurements were within the 95th percentile for their age, height, and gender. Patients were excluded if they had a comorbid psychiatric diagnosis, a history or family history of cardiac disease that the investigator judged to put the patient at risk, or were taking a CYP3A4- or P450-affecting agent. Informed consent was given by the patient's parent or guardian.

Patients received a single 2 mg dose on day 1 followed by repeated 2mg qd (once daily) doses on Days 9-15, repeated 3mg qd doses on Days 16-23, and repeated 4mg qd doses on Days 24-29. The dose was then titrated down to 3 mg on days 30 to 32, and 2 mg on days 33 to 35. Pharmacokinetic assessments were made after the single 2mg dose and after 6 days of repeated 2mg and 4mg doses.

**Results:** All 28 enrolled patients completed the trial. Demographic data are provided in the following table.

**Demographic information**

| | Children (n = 14) | Adolescents (n = 14) | Total (N = 28) |
|---|---|---|---|
| Age, mean (SD), y | 9.3 ± 1.82 | 14.2 ± 1.05 | 11.8 ± 2.90 |
| Gender | | | |
| Male, % | 50 | 85.7 | 67.9 |
| Female, % | 50 | 14.3 | 32.1 |
| Race | | | |
| White, % | 64.3 | 85.7 | 75 |
| African-American % | 28.6 | 7.1 | 17.9 |
| Other, % | 7.1 | 7.1 | 7.1 |
| Weight, mean (SD) | 76.6 ± 23.78 | 125.9 ± 20.87 | 101.2 ± 33.34 |
| Height, mean (SD) | 54.3 ± 4.59 | 65.1 ± 3.93 | 59.7 ± 6.91 |
| Body mass index, mean (SD), kg/m² | 17.8 ± 2.60 | 20.8 (2.33) | 19.3 ± 2.85 |

Pharmacokinetic findings are summarized in the following tables. Summary of pharmacokinetic parameters for guanfacine after oral administration of 2mg on Day 1 to children (6-12 years) and adolescent (13-17 years) ADHD patients

| Parameter* | Children (6-12 yrs) (N = 14) | Adolescents (13-17 yrs) (N=14) |
|---|---|---|
| Cₘₐₓ (ng/mL) | 2.55 ± 1.03 | 1.69 ± 0.43 |
| Tₘₐₓ (h) | 4.98 | 4.96 |
| AUC₀₋₁ (h·ng/mL) | 56.9 ± 22.0 | 42.7 ± 12.9 |
| AUC_{0-∞} (h·ng/mL) | 65.2 ± 23.9 | 47.3 ± 13.7 |
| λ_{z} (h⁻¹) | 0.0496 ± 0.0093 | 0.0428 ± 0.0153 |
| T_{½} (h) | 14.4 ± 2.39 | 17.9 ± 5.77 |
| CL/F (m L/min) | 612 ± 252 | 815 ±214 |
| V_{z}/F (L) | 766 ±380 | 1,239 ± 432 |

Summary of pharmacokinetic parameters for guanfacine on Day 14 after oral administration of 2mg once daily to children (6-12 years) and adolescent (13-17 years) ADHD patients.

| Parameter* | Children (6-12 yrs) (N = 14) | Adolescents (13-17 yrs) (N = 14) |
|---|---|---|
| Cₘₐₓ (ng/mL) | 4.39 ± 1.66 | 2.86 ± 0.77 |
| Tₘₐₓ (h) | 4.98 | 4.53 |
| AUC₀₋₂₄ (h·ng/mL) | 70.0 ±28.3 | 48.2 ± 16.1 |
| CL/F(mL/min) | 552 ± 215 | 826 ± 486 |

Summary of pharmacokinetic parameters for guanfacine on Day 28 after oral administration of 4mg once daily to children (6-12 years) and adolescent (13-17 years) ADHD patients.

| Parameter* | Children (6-12 yrs) (N = 14) | Adolescents (13-17 yrs) (N = 14) |
|---|---|---|
| Cₘₐₓ (ng/mL) | 10.1 ± 7.09 | 7.01 ± 1.53 |
| Tₘₐₓ (h) | 5.02 | 4.97 |
| AUC₀₋₂₄ (h·ng/mL) | 162 ± 116 | 117 ± 28.4 |
| CL/F (mL/min) | 522 ± 212 | 607 ± 166 |

There were no discontinuations due to adverse events (AEs). No clinically significant abnormalities were observed on ECGs, hematologic parameters, or serum chemistries. Most AEs were mild to moderate in severity. The only AE to occur in ≥10% of subjects and considered possibly or probably due to treatment was somnolence (93%); only 6 subjects (21%) were judged to have severe somnolence.

Conclusions: The pharmacokinetics of guanfacine are linear after oral administration of single 2mg doses and multiple 2mg and 4mg doses in both children (6-12 years) and adolescent (13-17 years) ADHD patients. Plasma concentrations and concentration-related pharmacokinetic parameters in children (6-12 years) are higher than those in adolescents (13-17 years) and, regardless of age group, are higher in female patients than in male patients. This is most likely due to the higher body weight in the adolescents compared to children and in males compared to females, regardless of age group. AEs were generally mild to moderate in severity and did not lead to any discontinuations. Controlled-release guanfacine appears to be a safe option for the treatment of ADHD in children and adolescents.

### Example 3

**Objectives:** The study was designed to assess the bioequivalence of guanfacine 2 mg and 4 mg tablets from table 2 and to investigate the dose proportionality of 1 mg, 2 mg, and 4 mg doses.

**Methods:** This was a Phase I, randomized, open-label, single-dose, five period, four-treatment crossover design with a separate lead-in period.

Forty-nine (49) healthy adult volunteers completed the study. During period 1, all subjects received a single 1 mg dose administered after and overnight fast. Prior to period 2, subjects were randomly assigned to one of four sequence groups, with 13 subjects per sequence. During treatment periods 2-5, subjects received their assigned treatment of 2 mg or 4 mg. All subjects were to receive two single doses of 2 mg tablets and two single doses of 4 mg tablets during the course of the study. Doses during periods 2-5 were also administered after an overnight fast.

| | |
|---|---|
| Demographics: Gender: | Males: 28; Females: 24 |
| | Race: White: 7; Black: 5; Hispanic: 40; Other: 0 |
| | Overall mean age in years: 33. Age range 18-55 inclusive |

### Results: A summary of the pharmacokinetic data is shown below:

Summary of PK parameters for guanfacine after single oral administration of 1 mg, 2 mg and 4 mg tablets.

| Parameter*† | 1 mg (N=52) | 2 mg (#1) (N=50) | 2 mg (#2) (N=50) | 4 mg (#1) (N=49) | 4 mg (#2) (N=49) |
|---|---|---|---|---|---|
| Cₘₐₓ (ng/mL) | 0.98±0.26 | 1.59 ± 0.49 | 1.54 ± 0.53 | 3.58 ±1.39 | 3.59 ± 1.40 |
| tₘₐₓ (h) | 6.00 | 6.00 | 6.00 | 5.01 | 6.00 |
| AUC ₀₋ₜ (h·ng/mL) | 29.3 ± 8.84 | 55.0 ± 18.0 | 54.0 ± 17.6 | 120 ± 41.5 | 119 ± 43.4 |
| AUC _{0-∞} (h·ng/mL) | 32.4 ± 8.78 | 58.1 ± 18.8 | 57.8 ± 19.1 | 125 ± 46.0 | 123 ± 44.6 |
| λ_{z} (h⁻¹) | 0.0416 ± 0.0088 | 0.0439 ± 0.0075 | 0.0434 ± 0.0086 | 0.0434 ± 0.0098 | 0.0448 ± 0.0089 |
| t_{½} (h) | 17.5 ± 3.83 | 16.4 ± 3.46 | 16.7 ± 4.12 | 17.1 ± 5.51 | 16.2 ± 4.15 |
| CL/F (mL/min) | 560 ± 194 | 647 ± 253 | 638 ± 206 | 599 ± 213 | 638 ± 331 |
| V_{z}/F (L) | 823 ± 249 | 894 ± 328 | 889 ± 241 | 854 ± 294 | 866 ± 393 |

**Conclusions:** The pharmacokinetics of guanfacine were reasonably linear over doses of 1 mg, 2 mg, and 4 mg although mean values for Cₘₐₓ and AUC₀₋ₗₐₛₜ and AUC_{inf} increased somewhat less than two fold between the 1 mg and 2 mg doses.
The 2 mg and 4 mg guanfacine tablets from two different data sets, i.e., (#1) and (#2), were bioequivalent to their respective strength tablets.

There were no deaths or serious adverse events.

### Example 4

**Objectives:** The objective of this study was to investigate the bioequivalence of guanfacine 2.5 mg tablets versus guanfacine 2 mg tablets.

**Methods:** This Phase I study utilized a randomized, open-label, single-dose, three-treatment crossover design. Forty-eight (48) healthy adult volunteers completed the study. All subjects were randomly assigned to one of 6 treatment sequences and received a 2 mg and 2.5 mg tablet of guanfacine. During treatment periods 1-3, subjects received their assigned treatment (a single oral dose 2 mg or 2.5 mg after an overnight fast).

| | |
|---|---|
| Demographics: Gender: | Males: 14; Females: 34 |
| | Race: White: 10; Black: 1; Hispanic: 36; Other: 1 |
| | Overall mean age in years: 33 |

### Results: A summary of the pharmacokinetic data is shown below:

Summary of PK parameters for guanfacine after oral administration of single 2 mg and 2.5 mg doses to healthy adult subjects.

| Parameter | 2 mg | 2.5 mg (#1) | 2.5 mg (#2) |
|---|---|---|---|
| Cₘₐₓ (ng/mL) | 1.71 ± 0.56 | 2.26 ± 0.67 | 2.49 ± 0.93 |
| Tₘₐₓ (hr) | 6 | 5 | 6 |
| AUC₀₋ₗₐₛₜ (ng.hr/mL) | 59.8 ± 20.9. | 77.8 ± 30.4 | 81.3 ± 35.4 |
| AUC_{inf}(ng.hr/mL) | 64.2 ± 22.6 | 84.5 ± 34.2 | 85.0 ± 37.4 |
| λz (h⁻¹) | 0.043 ± 0.012 | 0.040 ± 0.012 | 0.046 ± 0.011 |
| T1/2 (hr) | 17.7 ± 5.8 | 19.0 ± 6.6 | 16.7 ± 7.4 |
| CL/F (m L/min) | 612 ± 303 | 598 ± 299 | 591 ± 283 |
| Vz/F (L) | 884 ± 382 | 915 ± 390 | 809 ± 406 |

| | | | |
|---|---|---|---|
| Arithmetic mean ± standard deviation except for Tₘₐₓ for which the median is reported. | | | |

There were no deaths.

Conclusions: When corrected for the difference in dose, the 2.5mg guanfacine tablets from the two data sets, i.e., (#1) and (#2), are each bioequivalent to the 2mg guanfacine tablets. The tablets appeared to be safe and generally well tolerated by the healthy male and female subjects in this study when administered in single oral 2mg and 2.5mg doses.

### Example 5

### Osmotically controlled Guanfacine extended Release Tablets

The primary pieces of equipment useful for manufacturing osmotic tablets are a 16 quart V-shaped blender equipped with an intensifier bar, pan coater capable of solvent coating, a 16 station rotary tablet press, and laser hole drilling system to create the orifice from which drug is released. All materials including osmagents (Xylitol, Maltrin, and Mannitol) are passed through a 20 mesh screen and charged into a 16 quart V-blender, with guanfacine sandwiched in the middle. The blend is charged into a polyethylene bag and then transferred to the hopper of a Stokes tablet press. The blend is compressed to the appropriate hardness for the necessary tablet weight. Tablet hardness is tested with a Schleuniger hardness tester. Tablets are coated in a pan coater with spray rate of 60-100 g/min or higher. The coating solution is prepared by dissolving about 5% cellulose Acetate, NF (National Formulary) in Acetone then adding 25-45% plasticizers such as TEC. A laser drilled hole is then placed on one side of the tablets to allow for drug release. The tablet achieves the desired PK profile.

The preceding examples can be repeated with similar success by substituting the generically or specifically described reactants and/or operating conditions of this invention for those used in the preceding examples.

## Claims

1. An oral dosage form comprising from 0.75% to 2% by weight of guanfacine or a pharmaceutically acceptable salt thereof, wherein said dosage form is effective for once a day administration, and wherein said dosage form also comprises from 25 % to 45 % by weight poly(methacrylic acid, ethyl acrylate).

2. An oral dosage form according to claim 1 wherein guanfacine or a pharmaceutically acceptable salt thereof is guanfacine hydrochloride.

3. An oral dosage form according to claim 1 or claim 2 comprising 25 % to 35 % poly(methacrylic acid, ethyl acrylate).

4. An oral dosage from according to any one of the preceding claims in the form of a tablet.

5. An oral dosage form according to claim 4 having a hardness of 4.0 to 11.0 kp (39.23 to 107.87N), preferably 4.5 to 9.5 kp (44.13 to 93.16N), more preferably 4.5 to 8.0kp (44.13 to 78.45N), measured using a Schleuniger tablet hardness tester.

6. An oral dosage form according to any one of the preceding claims comprising:
Guanfacine Hydrochloride: 0.76 to 0.88 % weight/weight;
Hypromellose 2208: 13.34% weight/weight;
High density silicified microcrystatline cellulose: 15.38 to 17.33 % weight/weight;
Lactose monohydrate, povidone, crospovidone granulated blend:16.91 to 16.94 % weight/weight;
Methacrylic acid copolymer: 33.33 to 34.61% weight/weight;
Furaric acid atomized glyceryl behenate: 5.00% weight/weight; and
Atomized glyeryl behenate: 13.33 to 13.85 % weight/weight.

7. An oral dosage form according to claim 6 comprising:
Guanfacine Hydrochloride: 0.76 % weight/weight;
Hypromellose 2208:13.34 % weight/weight;
High density silicified microcrystalline cellulose: 17.33 % weight/weight;
Lactose monolihydrate, povidone, crospovidone granulated blend: 16.91% weight/weight;
Methacrylic acid copolymer: 33.33 % weight/weight;
Furaric acid atomized glyceryl behenate: 5.00 % weight/weight; and
Atomized glyeryl behenate: 13.33 % weight/weight.

8. An oral dosage form according to claim 6 or claim 7, in a tablet form and having a weight of 150 mg.

9. An oral do dosage form according to claim 6 or claim 7, in a tablet form and having a weight of 300 mg.

10. An oral dosage form according to any one of claims 1 to 5 comprising:
Guanfacine Hydrochloride: 1.27 to 1.42 % weight/weight;
Hypromellose 2208:13.34% weight/weight;
High density silicified microcrystalline cellulose: 17.10 to 17.33 % weight/weight;
Lactose monohydrate, povidone, crospovidone granulated blend: 15.90 to 15.99 % weight/weight;
Methacrylic acid copolymer: 33.33 % weight/weight;
Furaric acid atomized glyceryl behenate: 5.00 % weight/weight;
Atomized glyeryl behenate: 13.33 % weight/weight; and
Pigmented blend powder: 0.50 % weight/weight.

11. An oral dosage form according to claim 10 comprising:
Guanfacine Hydrochloride: 1.27 % weight/weight;
Hypromellose 2208: 13.34 % weight/weight;
High density silicified microcrystalline cellulose: 17.33 % weight/weight;
Lactose monohydrate, povidone, crospovidone granulated blend: 15.90 % weight/weight;
Methacrylic acid copolymer: 33.33 % weight/weight;
Furaric acid atomized glyceryl behenate: 5.00 % weight/weight;
Atomized glyeryl behenate: 13.33 % weight/weight; and
Pigmented blend powder: 0.50 % weight/weight.

12. An oral dosage form according to claim 10 or claim 11, in a tablet form and having a weight of 225 mg.

13. An oral dosage form according to any one of claims 1 to 5 comprising:
Guanfacine Hydrochloride: 1.71 to 1.90 % weight/weight;
Hypromellose 2208: 10.00 % weight/weight;
High density silicified microcrystalline cellulose: 21.04 to 21.39 % weight/weight;
Lactose monohydrate, povidone, crospovidone granulated blend: 21.40 to 21.55 % weight/weight;
Methacrylic acid copolymer: 25.00 % weight/weight;
Furaric acid atomized glyceryl behenate: 5.00 % weight/weight;
Atomized glyeryl behenate: 15.00% weight/weight; and
Pigmented blend powder: 0.50 % weight/weight.

14. An oral dosage form according to claim 10 comprising:
Guanfacine Hydrochloride: 1.71 % weight/weight;
Hypromellose 2208: 10.00 % weight/weight;
High density silicified microcrystalline cellulose: 21.39 % weight/weight;
Lactose monohydrate, povidone, crospovidone granulated blend: 21.40 % weight/weight;
Methacrylic acid copolymer: 25.00 % weight/weight;
Furaric acid atomized glyceryl behenate: 5.00 % weight/weight;
Atomized glyeryl behenate: 15.00 % weight/weight; and
Pigmented blend powder: 0.50 % weight/weight.

15. An oral dosage form according to claim 13 or claim 14, in a tablet form and having a weight of 200 mg.

16. An oral dosage form according to claim 13 or claim 14, in a tablet form and having a weight of 233.33 mg.

17. An oral dosage form according to claim 13 or claim 14, in a tablet form and having a weight of 266.66 mg.

18. A packaged pharmaceutical composition comprising a container holding an oral dosage form according to any one of claims 1 to 17, and instructions for use for an indication for guanfacine.

19. An oral dosage form according to any one of preceding claims for use in the treatment of a disorder selected from Attention Deficient Hyperactivity Disorder (ADHD), hypertension, heroin withdrawal and sleep disorders.

## Patentansprüche

1. Orale Darreichungsform, die von 0,75 bis 2 Gewichts% Guanfacin oder ein pharmazeutisch akzeptables Salz davon beinhaltet, wobei die Dareichungsform für einmal tägliche Gabe effektiv ist und wobei die Dareichungsform auch von 25 bis 45 Gewichts% Poly(methacrylsäure, -Ethylacrylat) beinhaltet.

2. Orale Darreichungsform gemäß Anspruch 1, wobei Guanfacin oder ein pharmazeutisch akzeptables Salz davon Guanfacinhydrochlorid ist.

3. Orale Darreichungsform gemäß Anspruch 1 oder Anspruch 2, die 25 % bis 35 % Poly(methacrylsäure, -Ethylacrylat) beinhaltet.

4. Orale Darreichungsform gemäß einem der vorhergehenden Ansprüche in Form einer Tablette.

5. Orale Darreichungsform gemäß Anspruch 4, die eine Härte von 4,0 bis 11,0 kp (39,23 bis 107,87 N), bevorzugt 4,5 bis 9,5 kp (44,13 bis 93,16 N), besonders bevorzugt 4,5 bis 8,0 kp (44,13 bis 78,45 N), unter Verwendung eines Schleuniger Tablettenhärteprüfgeräts gemessen, aufweist.

6. Orale Darreichungsform gemäß einem der vorhergehenden Ansprüche, die Folgendes beinhaltet:
Guanfacinhydrochlorid: 0,76 bis 0,88 % Gewicht/Gewicht;
Hypromellose 2208:13,34 Gewichts%;
Hochdichte verkieselte mikrokristalline Cellulose: 15,38 bis 17,33 % Gewicht/Gewicht;
Granuliertes Gemisch aus Lactose-Monohydrat, Povidon, Crospovidon: 16,91 bis 16,94 % Gewicht/Gewicht;
Methacrylsäure-Copolymer: 33,33 bis 34,61 % Gewicht/Gewicht;
Fumarsäure verdüstes Glycerylbehenat: 5,00 % Gewicht/Gewicht; und
verdüstes Glycerylbehenat: 13,33 bis 13,85 % Gewicht/Gewicht;

7. Orale Darreichungsform gemäß Anspruch 6, die Folgendes beinhaltet:
Guanfacinhydrochlorid: 0,76 % Gewicht/Gewicht;
Hypromellose 2208: 13,34 % Gewicht/Gewicht;
Hochdichte verkieselte mikrokristalline Cellulose: 17,33 % Gewicht/Gewicht;
Granuliertes Gemisch aus Lactose-Monohydrat, Povidon, Crospovidon: 16,91 % Gewicht/Gewicht;
Methacrylsäure-Copolymer: 33,33 % Gewicht/Gewicht;
Fumarsäure verdüstes Glycerylbehenat: 5,00 % Gewicht/Gewicht; und
verdüstes Glycerylbehenat: 13,33 % Gewicht/Gewicht;

8. Orale Darreichungsform gemäß Anspruch 6 oder Anspruch 7 in Tablettenform und mit einem Gewicht von 150 mg.

9. Orale Darreichungsform gemäß Anspruch 6 oder Anspruch 7 in Tablettenform und mit einem Gewicht von 300 mg.

10. Orale Darreichungsform gemäß einem der Ansprüche 1 bis 5, die Folgendes beinhaltet:
Guanfacinhydrochlorid: 1,27 bis 1,42 % Gewicht/Gewicht;
Hypromellose 2208: 13,34% Gewicht/Gewicht;
Hochdichte verkieselte mikrokristalline Cellulose: 17,10 bis 17,33 % Gewicht/Gewicht;
Granuliertes Gemisch aus Lactose-Monohydrat, Povidon, Crospovidon: 15,90 bis 15,99 % Gewicht/Gewicht;
Methacrylsäure-Copolymer: 33,33 % Gewicht/Gewicht;
Fumarsäure verdüstes Glycerylbehenat: 5,00 % Gewicht/Gewicht;
Verdüstes Glycerylbehenat: 13,33 % Gewicht/Gewicht; und
pigmentiertes Gemischpulver: 0,50 % Gewicht/Gewicht;

11. Orale Darreichungsform gemäß Anspruch 10, die Folgendes beinhaltet:
Guanfacinhydrochlorid: 1,27 % Gewicht/Gewicht;
Hypromellose 2208: 13,34 % Gewicht/Gewicht;
Hochdichte verkieselte mikrokristalline Cellulose: 17,33 % Gewicht/Gewicht;
Granuliertes Gemisch aus Lactose-Monohydrat, Povidon, Crospovidon: 15,90 % Gewicht/Gewicht;
Methacrylsäure-Copolymer: 33,33 % Gewicht/Gewicht;
Fumarsäure verdüstes Glycerylbehenat: 5,00 % Gewicht/Gewicht;
verdüstes Glycerylbehenat: 13,33 % Gewicht/Gewicht; und
pigmentiertes Gemischpulver: 0,50 % Gewicht/Gewicht;

12. Orale Darreichungsform gemäß Anspruch 10 oder Anspruch 11 in Tablettenform und mit einem Gewicht von 225 mg.

13. Orale Darreichungsform gemäß einem der Ansprüche 1 bis 5, die Folgendes beinhaltet:
Guanfacinhydrochlorid: 1,71 bis 1,90 % Gewicht/Gewicht;
Hypromellose 2208: 10,00 % Gewicht/Gewicht;
Hochdichte verkieselte mikrokristalline Cellulose: 21,04 bis 21,39 % Gewicht/Gewicht;
Granuliertes Gemisch aus Lactose-Monohydrat, Povidon, Crospovidon: 21,40 bis 21,55 % Gewicht/Gewicht;
Methacrylsäure-Copolymer: 25,00 % Gewicht/Gewicht;
Fumarsäure verdüstes Glycerylbehenat: 5,00 % Gewicht/Gewicht;
verdüstes Glycerylbehenat: 15,00 % Gewicht/Gewicht; und
pigmentiertes Gemischpulver: 0,50 % Gewicht/Gewicht;

14. Orale Darreichungsform gemäß Anspruch 10, die Folgendes beinhaltet:
Guanfacinhydrochlorid: 1,71 % Gewicht/Gewicht;
Hypromellose 2208: 10,00 % Gewicht/Gewicht;
Hochdichte verkieselte mikrokristalline Cellulose: 21,39 % Gewicht/Gewicht;
Granuliertes Gemisch aus Lactose-Monohydrat, Povidon, Crospovidon: 21,40 % Gewicht/Gewicht;
Methacrylsäure-Copolymer: 25,00 % Gewicht/Gewicht;
Fumarsäure verdüstes Glycerylbehenat: 5,00 % Gewicht/Gewicht;
verdüstes Glycerylbehenat: 15,00 % Gewicht/Gewicht; und
pigmentiertes Gemischpulver: 0,50 % Gewicht/Gewicht;

15. Orale Darreichungsform gemäß Anspruch 13 oder Anspruch 14 in Tablettenform und mit einem Gewicht von 200 mg.

16. Orale Darreichungsform gemäß Anspruch 13 oder Anspruch 14 in Tablettenform und mit einem Gewicht von 233,33 mg.

17. Orale Darreichungsform gemäß Anspruch 13 oder Anspruch 14 in Tablettenform und mit einem Gewicht von 266,66 mg.

18. Verpackte pharmazeutische Zusammensetzung, die einen Behälter beinhaltet, der eine orale Darreichungsform gemäß einem der Ansprüche 1 bis 17 und Anweisungen zur Verwendung für eine Indikation für Guanfacin enthält.

19. Orale Darreichungsform gemäß einem der vorhergehenden Ansprüche zur Verwendung in der Behandlung einer Störung ausgewählt aus Aufmerksamkeitsdefizit-/Hyperaktivitätsstörung (ADHD, Attention Deficient Hyperactivity Disorder), Hypertonie, Heroinentzugs- und Schlafstörungen.

## Revendications

1. Forme posologique orale comprenant de 0,75% à 2% en poids de guanfacine ou un sel pharmaceutiquement acceptable de celle-ci, dans laquelle ladite forme posologique est efficace pour une administration une fois par jour, et dans laquelle ladite forme posologique comprend en outre de 25% à 45% en poids de poly (acide méthacrylique, acrylate d'éthyle).

2. Forme posologique orale selon la revendication 1, où la guanfacine ou un sel pharmaceutiquement acceptable de celle-ci est l'hydrochlorure de guanfacine.

3. Forme posologique orale selon la revendication 1 ou la revendication 2 comprenant de 25% à 35% de poly(acide méthacrylique, acrylate d'éthyle).

4. Forme posologique orale selon l'une quelconque des revendications précédentes sous forme de comprimé.

5. Forme posologique orale selon la revendication 4, ayant une dureté de 4,0 à 11,0 kp (39,23 à 107,87N), de préférence 4,5 à 9,5 kp (44,13 à 93,16N), plus préférablement 4,5 à 8,0kp (44,13 à 78,45N), mesurée en utilisant un testeur de dureté de comprimés Schleuniger.

6. Forme posologique orale selon l'une quelconque des revendications précédentes comprenant :
Hydrochlorure de guanfacine : 0,76 à 0,88% poids/poids ;
Hypromellose 2208 : 13,34% poids/poids ;
Cellulose microcristalline silicifiée haute densité : 15,38 à 17,33% poids/poids ;
Mélange granulé de monohydrate de lactose, povidone, crospovidone : 16,91 à 16,94% poids/poids ;
Copolymère d'acide acrylique : 33,33 à 34,61% poids/poids ;
Béhénate de glycéryl atomisé d'acide furarique : 5,00% poids/poids ; et
Béhémate de glycéryl atomisé : 13,33 à 13,85% poids/poids.

7. Forme posologique orale selon la revendication 6 comprenant :
Hydrochlorure de guanfacine : 0,76% poids/poids ;
Hypromellose 2208 : 13,34% poids/poids ;
Cellulose microcristalline silicifiée haute densité : 17,33% poids/poids ;
Mélange granulé de monohydrate de lactose, povidone, crospovidone : 16,91% poids/poids ;
Copolymère d'acide acrylique: 33,33% poids/poids ;
Béhénate de glycérol atomisé d'acide furarique: 5,00% poids/poids; et béhénate de glycéryl atomisé : 13,33% poids/poids.

8. Forme posologique orale selon la revendication 6 ou la revendication 7, sous forme de comprimé et ayant un poids de 150 mg.

9. Forme posologique orale selon la revendication 6 ou la revendication 7, sous forme de comprimé et ayant un poids de 300 mg.

10. Forme posologique orale selon l'une quelconque des revendications 1 à 5 comprenant :
Hydrochlorure de guanfacine : 1,27 à 1,42% poids/poids ;
Hypromellose 2208 :13,34% poids/poids ;
Cellulose microcristalline silicifiée haute densité : 17,10 à 17,33% poids/poids ;
Mélange granulé de monohydrate de lactose, povidone, crospovidone : 15,90 à 15,99% poids/poids ;
Copolymère d'acide acrylique : 33,33% poids/poids ;
Béhénate de glycérol atomisé d'acide furarique : 5,00% poids/poids ;
Béhémate de glycéryl atomisé : 13,33% poids/poids ; et
Poudre de mélange pigmenté: 0,50% poids/poids.

11. Forme posologique orale selon la revendication 10 comprenant :
Hydrochlorure de guanfacine : 1,27% poids/poids ;
Hypromellose 2208 : 13,34% poids/poids ;
Cellulose microcristalline silicifiée haute densité : 17,33% poids/poids ;
Mélange granulé de monohydrate de lactose, povidone, crospovidone : 15,90% poids/poids ;
Copolymère d'acide acrylique : 33,33% poids/poids ;
Béhénate de glycérol atomisé d'acide furarique : 5,00% poids/poids ;
Béhémate de glycéryl atomisé : 13,33% poids/poids ; et
Poudre de mélange pigmenté : 0,50% poids/poids.

12. Forme posologique orale selon la revendication 10 ou la revendication 11, sous forme de comprimé et ayant un poids de 225 mg.

13. Forme posologique orale selon l'une quelconque des revendications 1 à 5 comprenant :
Hydrochlorure de guanfacine : 1,71 à 1,90% poids/poids ;
Hypromellose 2208 : 10,00% poids/poids ;
Cellulose microcristalline silicifiée haute densité : 21,04 à 21,39% poids/poids ;
Mélange granulé de monohydrate de lactose, povidone, crospovidone : 21,40 à 21,55% poids/poids ;
Copolymère d'acide acrylique : 25,00% poids/poids ;
Béhénate de glycérol atomisé d'acide furarique : 5,00% poids/poids ;
Béhémate de glycéryl atomisé : 15,00% poids/poids ; et
Poudre de mélange pigmenté: 0,50% poids/poids.

14. Forme posologique orale selon la revendication 10 comprenant :
Hydrochlorure de guanfacine : 1,71% poids/poids ;
Hypromellose 2208 : 10,00% poids/poids ;
Cellulose microcristalline silicifiée haute densité : 21,39% poids/poids ;
Mélange granulé de monohydrate de lactose, povidone, crospovidone : 21,40% poids/poids ;
Copolymère d'acide acrylique : 25,00% poids/poids ;
Béhénate de glycérol atomisé d'acide furarique : 5,00% poids/poids ;
Béhémate de glycéryl atomisé : 15,00% poids/poids ; et
Poudre de mélange pigmenté : 0,50% poids/poids.

15. Forme posologique orale selon la revendication 13 ou la revendication 14, sous forme de comprimé et ayant un poids de 200 mg.

16. Forme posologique orale selon la revendication 13 ou la revendication 14, sous forme de comprimé et ayant un poids de 233,33 mg.

17. Forme posologique orale selon la revendication 13 ou la revendication 14, sous forme de comprimé et ayant un poids de 266,66 mg.

18. Composition pharmaceutique conditionnée comprenant un récipient contenant une forme posologique orale conforme à l'une quelconque des revendications 1 à 17, et instructions d'utilisation selon une indication pour la guanfacine.

19. Forme posologique orale conforme à l'une des revendications précédentes pour utilisation dans le traitement d'un trouble sélectionné parmi le trouble d'hyperactivité avec déficit de l'attention (THDA), l'hypertension, le sevrage de l'héroïne et les troubles du sommeil.
